# EUROPEAN PATENT APPLICATION

(11) **EP 1 508 576 A1**
(43) Date of publication of application: **23.02.2005**
(21) Application number: 03102598.4
(22) Date of filing: 20.08.2003
(51) Int. Cl.: C07K 16/00, C12N 5/10

(54) **Efficient production of IgA in recombinant mammalian cells**

(71) Applicant: Crucell Holland B.V., 2333 CN Leiden (NL)
(72) Inventor: JONES, David, Halford, Ashton, Tooting London 5W17 9SB (GB); BOUT, Abraham, 2751 XL, Moerkapelle (NL)

(57) **Abstract**

The invention provides an immortalized human retina cell expressing E1A and E1B proteins of an adenovirus, wherein said cell comprises recombinant nucleic acid encoding an IgA molecule in expressible format. The invention also provides a method for recombinant production of an IgA molecule, said method comprising culturing a cell according to the invention and expressing said recombinant nucleic acid encoding an IgA.

SEQUENCE LISTING

<110>  CRUCELL HOLLAND B.V.
       Jones, David H.A.
       Bout, Abraham

<120>  Efficient production of IgA in recombinant mammalian cells

<130>  IgA

<160>  8

<170>  PatentIn version 3.2

<210>  1
<211>  2022
<212>  DNA
<213>  Artificial

<220>
<223>  genomic DNA encoding heavy chain of anti -EpCAM IgA

<220>
<221>  startcod on
<222>  (1)..(3)

<220>
<221>  stopcodon
<222>  (2020)..(2022)

<400>  1
atggcatgcc ctggcttcct gtgggcactt gtgatctcca cctgtcttga attttccatg      60

gcccaggtgc agctggtgca gtctggggct gaggtgaaga agcctgggtc ctcggtgagg     120

gtctcctgca aggcttctgg aggcaccttc  agcagctatg ctatcagctg ggtgcgacag     180

gcccctggac aagggcttga gtggatggga gggatcatcc ctatctttgg tacagcaaac     240

tacgcacaga agttccaggg cagagtcacg attaccgcgg acgaatccac gagcacagcc     300

tacatggagc tgagcagcct gagatctgag gacacggctg tgtattactg tgcaagagac      360

ccgtttcttc actattgggg ccaaggtacc ctggtcaccg tctcgacagg tgagtgcggc     420

cgctctgtgc tgggttcctc cagtatagag gagaggcagg cacagactgt cctcctgggg     480

acatggcatg agggccgcgt cctcacagtg cattctgtgt tccagcatcc ccgaccagcc     540

ccaaggtctt cccgctgagc ctctg cagca cccagccaga tgggaacgtg gtcatcgcct     600

gcctggtcca gggcttcttc ccccaggagc cactcagtgt gacctggagc gaaagcggac     660

agggcgtgac cgccagaaac ttcccaccca gccaggatgc ctccggggac ctgtacacca     720

cgagcagcca gctgaccctg ccggccacac agtgcctagc cggcaagtcc gtgaca tgcc     780

acgtgaagca ctacacgaat cccagccagg atgtgactgt gccctgccca ggtcagaggg     840

caggctgggg agtggggcgg ggccaccccg tcgtgccctg acactgcgcc tgcacccgtg     900

ttccccacag ggagccgccc cttcactcac accagagtgg accgcgggcc gagccccagg     960

aggtggtggt ggacaggcca  ggaggggcga ggcgggggca tggggaagca tgtgctgacc    1020

agctcaggcc atctctccac tccagttccc tcaactccac ctaccccatc tccctcaact    1080

ccacctaccc catctccctc atgctgccac ccccgactgt cactgcaccg accggccctc    1140

gaggacctgc tcttaggttc agaagcgaac ctcacgtgca cactgaccgg  cctgagagat    1200

gcctcaggtg tcaccttcac ctggacgccc tcaagtggga agagcgctgt tcaaggacca    1260

cctgaccgtg acctctgtgg ctgctacagc gtgtccagtg tcctgtcggg ctgtgccgag    1320

ccatggaacc atgggaagac cttcacttgc actgctgcct accccgagtc caagaccccg    1380

ctaaccgcca ccct ctcaaa atccggtggg tccagaccct gctcggggcc ctgctcagtg    1440

ctctggtttg caaagcatat tcctggcctg cctcctccct cccaatcctg ggctccagtg    1500

ctcatgccaa gtacagaggg aaactgaggc aggctgaggg gccaggacac agcccggggt    1560

gcccaccaga gcagaggggc tctctcatcc cctgcccagc cccct gacct ggctctctac    1620

cctccaggaa acacattccg gcccgaggtc cacctgctgc cgccgccgtc ggaggagctg 1680

gccctgaacg agctggtgac gctgacgtgc ctggcacgtg gcttcagccc caaggatgtg 1740

ctggttcgct ggctgcaggg gtcacaggag ctgccccgcg agaagtacct gacttgggca 1800

tcccggcagg agcccagcca gggcaccacc accttcgctg tgaccagcat actgcgcgtg 1860

gcagccgagg actggaagaa gggggacacc ttctcctgca tggtgggcca cgaggccctg 1920

ccgctggcct tcacacagaa gaccatcgac cgcttggcgg gtaaacccac ccatgtcaat 1980

gtgtctgttg tcatggcgga ggtggacggc acctgctact ga 2022

<210> 2
<211> 922
<212> DNA
<213> Artificial

<220>
<223> genomic DNA encoding light chain of anti -EpCAM IgA

<220>
<221> startcodon
<222> (1)..(3)

<220>
<221> stopcodon
<222> (920)..(922)

<400> 2
atggcatgcc ctggcttcct gtgggcactt gtgatctcca cctgtcttga attttccatg 60

gctgaaattg agctcactca gtctccactc tccctgcccg tcacccctgg agagccggcc 120

tccatctcct gcaggtctag tcagagcctc ctgcatagta atggatacaa ctatttggat 180

tggtacctgc agaagccagg gcagtctcca cagctcctga tctat ttggg ttctaatcgg 240

gcctccgggg tccctgacag gttcagtggc agtggatcag gcacagattt tacactgaaa 300

atcagcagag tggaggctga ggatgttggg gtttattact gcatgcaagc tctacaaact 360

ttcactttcg gccctgggac caaggtggag atcaaacgta agtgcacttt gcggccgcta 420

ggaagaaact caaaacatca agattttaaa tacgcttctt ggtctccttg ctataattat 480

ctgggataag catgctgttt tctgtctgtc cctaacatgc cctgtgatta tccgcaaaca 540

acacacccaa gggcagaact ttgttactta aacaccatcc tgtttgcttc tttcctcagg 600

aactgtggct gcaccatctg tcttcatctt cccgccatct gatgagcagt tgaaatctgg 660

aactgcctct gttgtgtgcc tgctgaataa cttctatccc agagaggcca aagtacagtg 720

gaaggtggat aacgccctcc aatcgggtaa ctcccaggag agtgtcacag agcaggacag 780

caaggacagc acctacagcc tcagcagcac cctgacgctg agcaaagcag actacgagaa 840

acacaaagtc tacgcctgcg aagtcaccca tcagggcctg agctcgcccg tcacaaagag 900

cttcaacagg ggagagtgtt ag                                                922

<210>  3
<211>  489
<212>  PRT
<213>  Artificial

<220>
<223>  amino acid sequence anti -EpCAM IgA heavy chain

<220>
<221>  leader peptide
<222>  (1)..(21)

<220>
<221>  VH region
<222>  (22)..(136)

<220>
<221>  CH1 region
<222>  (137)..(238)

<220>
<221>  CH2 region
<222>  (239)..(359)

<220>
<221>  CH3 region
<222>  (360)..(489)

<400>  3

Met Ala Cys Pro Gly Phe Leu Trp Ala Leu Val Ile Ser Thr Cys Leu
1               5                   10                  15

Glu Phe Ser Met Ala Gln Val Gln Leu Val Gln Ser Gly Ala Glu Val
            20                  25                  30

Lys Lys Pro Gly Ser Ser Val Arg Val Ser Cys Lys Ala Ser Gly Gly
        35                  40                  45

Thr Phe Ser Ser Tyr Ala Ile Ser Trp Val Arg Gln Ala Pro Gly Gln
    50                  55                  60

Gly Leu Glu Trp Met Gly Gly Ile Ile Pro Ile Phe Gly Thr Ala Asn
65                  70                  75                  80

Tyr Ala Gln Lys Phe Gln Gly Arg Val Thr Ile Thr Ala Asp Glu Ser
                85                  90                  95

Thr Ser Thr Ala Tyr Met Glu Leu Ser Ser Leu Arg Ser Glu Asp Thr
            100                 105                 110

Ala Val Tyr Tyr Cys Ala Arg Asp Pro Phe Leu His Tyr Trp Gly Gln
        115                 120                 125

Gly Thr Leu Val Thr Val Ser Thr Ala Ser Pro Thr Ser Pro Lys Val
    130                 135                 140

Phe Pro Leu Ser Leu Cys Ser Thr Gln Pro Asp Gly Asn Val Val Ile
145                 150                 155                 160

Ala Cys Leu Val Gln Gly Phe Phe Pro Gln Glu Pro Leu Ser Val Thr
                165                 170                 175

Trp Ser Glu Ser Gly Gln Gly Val Thr Ala Arg Asn Phe Pro Pro Ser
            180                 185                 190

Gln Asp Ala Ser Gly Asp Leu Tyr Thr Thr Ser Ser Gln Leu Thr Leu
        195                 200                 205

Pro Ala Thr Gln Cys Leu Ala Gly Lys Ser Val Thr Cys His Val Lys
210 215 2 20

His Tyr Thr Asn Pro Ser Gln Asp Val Thr Val Pro Cys Pro Val Pro
225                 230                 235                 240

Ser Thr Pro Pro Thr Pro Ser Pro Ser Thr Pro Pro Thr Pro Ser Pro
                245                 250                 255

Ser Cys Cys His Pro Arg Leu Ser Leu His Arg Pro Ala Leu Glu Asp
            260                 265                 270

Leu Leu Leu Gly Ser Glu Ala Asn Leu Thr Cys Thr Leu Thr Gly Leu
        275                 280                 285

Arg Asp Ala Ser Gly Val Thr Phe Thr Trp Thr Pro Ser Ser Gly Lys
    290                 295                 300

Ser Ala Val Gln Gly Pro Pro Asp Arg Asp Leu Cys Gly Cys Tyr Ser
305                 310                 315                 320

Val Ser Ser Val Leu Ser Gly Cys Ala Glu Pro Trp Asn His Gly Lys
                325                 330                 335

Thr Phe Thr Cys Thr Ala Ala Tyr Pro Glu Ser Lys Thr Pro Leu Thr
            340                 345                 350

Ala Thr Leu Ser Lys Ser Gly Asn Thr Phe Arg Pro Glu Val His Leu
        355                 360                 365

Leu Pro Pro Pro Ser Glu Glu Leu Ala Leu Asn Glu Leu Val Thr Leu
    370                 375                 380

Thr Cys Leu Ala Arg Gly Phe Ser Pro Lys Asp Val Leu Val Arg Trp
385                 390                 395                 400

Leu Gln Gly Ser Gln Glu Leu Pro Arg Glu Lys Tyr Leu Thr Trp Ala
                405                 410                 415

Ser Arg Gln Glu Pro Ser Gln Gly Thr Thr Thr Phe Ala Val Thr Ser
            420                 425                 430

Ile Leu Arg Val Ala Ala Glu Asp Trp Lys Lys Gly Asp Thr Phe Ser
        435                 440                 445

Cys Met Val Gly His Glu Ala Leu Pro Leu Ala Phe Thr Gln Lys Thr
    450                 455                 460

Ile Asp Arg Leu Ala Gly Lys Pro Thr His Val Asn Val Ser Val Va l
465 470 475 480

Met Ala Glu Val Asp Gly Thr Cys Tyr
485

<210>  4
<211>  239
<212>  PRT
<213>  Artificial

<220>
<223>  amino acid sequence anti -EpCAM IgA light chain

<220>
<221>  leader peptide
<222>  (1)..(21)

<220>
<221>  VL region
<222>  (22)..(132)

<220>
<221>  CL region
<222>  (133)..(239)

<400>  4

Met Ala Cys Pro Gly Phe Leu Trp Ala Leu Val Ile Ser Thr Cys Leu
1               5                   10                  15

Glu Phe Ser Met Ala Glu Ile Glu Leu Thr Gln Ser Pro Leu Ser Leu
            20                  25                  30

Pro Val Thr Pro Gly Glu Pro Ala Ser Ile Ser Cys Arg Ser Ser Gln
        35                  40                  45

Ser Leu Leu His Ser Asn Gly Tyr Asn Tyr Leu Asp Trp Tyr Leu Gln
    50                  55                  60

Lys Pro Gly Gln Ser Pro Gln Leu Leu Ile Tyr Leu Gly Ser Asn Arg
65                  70                  75                  80

Ala Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Val Gly Val Tyr
            100                 105                 110

Tyr Cys Met Gln Ala Leu Gln Thr Phe Thr Phe Gly Pro Gly Thr Lys
        115                 120                 125


    130                 135                 140


145                 150                 155                 160


                16 5                170                 175


            180                 185                 190


        195                 200                 205



210                 215                 220


225                 230                 235

<210>  5
<211>  38
<212>  DNA
<213>  Artificial

<220>
<223>  E001 forward primer for amplification of light chain

<400>  5
cctggcgcgc caccatggca tgccctggct tcctgtgg                              38

<210>  6
<211>  32
<212>  DNA
<213>  Artificial

<220>
<223>  E002 reverse primer for amplification of light chain

<400>  6
ccgggttaac taacactctc ccctgttgaa gc                                    32

<210>  7
<211>  39
<212>  DNA
<213>  Artificial

<220>
<223>  E003 forward primer for amplification of heavy chain

<400>  7
ggaggatccg ccaccatggc atgccctggc ttcctgtgg                             39

<210>  8
<211>  29
<212>  DNA
<213>  Artificial

<220>
<223>  P01 reverse primer for amplification of heavy chain

<400>  8
ggaccgctag ctcagtagca ggtgccgac                                        29

## Description

### FIELD OF THE INVENTION

The invention relates to the field of recombinant protein production. More in particular, the invention relates to the production of immunoglobulins of class A (IgA) in recombinant mammalian host cells.

### BACKGROUND OF THE INVENTION

Immunoglobulin molecules may be one of five classes based on amino acid sequence of the constant region of the molecule. These classes are IgG, IgM, IgA, IgD and IgE. Each class has different biological roles based on class-specific properties (Roitt, I., Brostoff, J., Make, D. (2001). Immunology. 6^{th} edition, pub. Mosby).

IgA is the first line of immune defence on mucosal surfaces where it is produced at approximately 3 - 5 g/day. Furthermore it is present in serum at around 3mg/ml, and as such is the most abundantly produced immunoglobulin. One IgA monomer comprises two heavy chains and two light chains. Light chains may be of the kappa or lambda class, and are shared with other immunoglobulin classes. The heavy chains of IgA consist of an N-terminal variable region, followed by a constant region, a hinge region, and then two additional constant regions. At the C-terminus there is a tail-piece which has a function in dimerisation of the molecule. IgA is found as two different isoforms, IgA1 and IgA2; the main difference in these isotypes is found in the hinge region, where IgA2 lacks a proline-rich region which in IgA1 is susceptible to bacterial protease activity. IgA1 is the predominant isotype in serum, whereas IgA2 is mainly produced by cells of the lamina propria where it is secreted on to mucosal surfaces. IgA2 is further divided into IgA(m)1 and IgA(m)2, depending on the nature of the disuphide linkage of heavy and light chains (Chintalacharuvu and Morrison, 1999).

Plasma IgA is found predominantly as a monomeric species, but mucosal IgA exists primarily in a dimeric form. A 15kDa J-chain binds to the molecule, stabilising the dimeric form, and upon secretion to the mucosal surface a 50 - 90kDa secretory component becomes complexed to the molecule. This protects the IgA from degradation on the luminal surface (Johansen et al., 2000, 2001).

The IgA1 hinge region includes three to five O-linked glycans, and the heavy chain includes two N-linked glycans (Mattu et al., 1998; Novak et al., 2000). The IgA2 subclass contains an additional two or three N-linked glycans. The role these glycans play in protein stability or effector function is not clear.

IgG1 does not bind neutrophils with a high affinity, and therefore is not proficient in neutrophil activation. IgA exerts its biological effects by a variety of mechanisms (Dechant and Valerius, 2001). It binds to the FcαRI receptor (CD89), which is expressed on neutrophils, eosinophils, monocytes/macrophages, though not on NK cells. Activation of FcαRI causes responses such as the oxidative burst and degranulation, but is also important in the triggering of phagocytosis. Neutrophils are the most prevalent lymphocytes in the blood and when activated are a powerful tool to combat infection. A second mechanism by which IgA exerts a response is by directly binding to and neutralising pathogens at mucosal surfaces, an effect enhanced by the dimeric nature of the IgA. Additionally, a mechanism by which antibodies are thought to eliminate target cells is by binding to, and often cross-linking, cell surface receptors (Ghetie et al., 1997; Tutt et al., 1998; Longo, 2002). This can lead to activation of signalling pathways resulting in arrest of cell growth or apoptosis. IgA does not appear to activate complement, but the other effector functions make it a potentially valuable adjunct to IgG in the clinical treatment of disease.

Few studies have been performed to test the value of IgA's as therapeutics (reviewed in Dechant and Valerius, 2001; Corthesy, 2002), in part because murine IgA does not bind the human FcαRI. Some studies have been performed which demonstrate IgA activity against infectious disease (Vidarsson et al., 2001; Spriel et al., 1999), and a few studies to look at cancer immunotherapy (Huls et al., 1999a; van Egmond et al., 2001).

There is also little data in the literature regarding IgA production in cell lines (reviewed in Chintalacharuvu and Morrison, 1999; Yoo et al., 2002). There is little information regarding productivity of such cells, but IgA production of up to 20 pg per cell per 24 hours has been described in CHO cells, though this included gene amplification (Berdoz et al, 1999). However, cells with high copy numbers of the transgene are reported to display instability upon propagation of the cells (Kim et al, 1998; Barnes et al, 2003). Glycan structures present on an IgA molecule produced in CHO cells has also been analysed, and it was found that CHO cells produced predominantly tri-antennary structures, whereas serum IgA contains mostly biantennary structures (Mattu et al., 1998). This may reflect a difference between human cells and CHO cell culture.

A need remains for a good production platform for recombinant IgA production, without the drawbacks associated with the existing platforms.

### BRIEF DESCRIPTION OF THE FIGURES

Fig. 1. Vector for expression of intact IgA. IgA-encoding regions, CMV promoters and the neomycin resistance marker are indicated.
Fig. 2. Reducing and non-reducing SDS-PAGE (silver-stained) of crude cell culture supernatant from a clone producing IgA. An IgA standard is also included.

### DESCRIPTION OF THE INVENTION

The characteristics of a platform for production of IgA would preferably include high IgA productivity and human-type glycosylation of the molecule. It is demonstrated herein that PER.C6™ cells are capable of efficiently producing and secreting recombinant IgA molecules. High levels of IgA are expressed from recombinant cells without the need for amplification of the copy number of the recombinant nucleic acid encoding the IgA.

In one aspect, the invention provides a cell expressing E1A and E1B proteins of an adenovirus, wherein said cell comprises recombinant nucleic acid encoding an IgA molecule in expressible format. In another aspect the invention provides a method for recombinant production of an IgA molecule, said method comprising: a) providing a cell expressing E1A and E1B proteins of an adenovirus, wherein said cell further comprises recombinant nucleic acid encoding an IgA molecule in expressible format; and b) culturing said cell and expressing said recombinant nucleic acid encoding an IgA. In another aspect, the invention provides for the use of a cell expressing E1A and E1B proteins of an adenovirus for recombinant expression of IgA molecules. In certain embodiments, said cells are derived from retina cells. In other embodiments, said cells are derived from primary cells, which have been immortalized by the expression of said E1A and E1B proteins. In other embodiments, said cells are human cells. In preferred embodiments the cells of the invention are immortalized human retina cells, more preferably PER.C6™ cells, or derived therefrom.

### DETAILED DESCRIPTION OF THE INVENTION

The present invention teaches that cells derived from human retina cells, which have been immortalized by introduction of E1 sequences from an adenovirus, are a good production platform for recombinant IgA molecules. A method for immortalization of embryonic retina cells has been described in US patent 5,994,128. Accordingly, an embryonic retina cell that has been immortalized with E1 sequences from an adenovirus can be obtained by that method. Other cells can expressing E1A and E1B of an adenovirus can be prepared accordingly. Preferably, the cells of the invention are human cells. In certain embodiments, said cells are derived from retina cells. Preferably said cells are derived from primary cells. A cell according to the inventioin expresses at least the E1A region of an adenovirus, and preferably also the E1B region. E1A protein protein has transforming activity, while E1B protein has anti-apoptotic activities. The cells of the invention therefore preferably express E1A and E1B proteins of an adenovirus. In preferred embodiments, such cells are derived from PER.C6™ cells, as deposited under ECACC number 96022940. Cells derived from a PER.C6™ cell according to the invention can be obtained by introduction of foreign genetic material encoding an IgA molecule into such PER.C6™ cells. Preferably said cells are from a stable clone that can be selected and propagated according to standard procedures known to the person skilled in the art. A culture of such a clone is capable of producing recombinant IgA molecules. Cells according to the invention preferably are able to grow in suspension culture in serum-free medium.

It has previously been shown that PER.C6™ cells can express intact human IgG (WO 00/63403), that such IgGs have human-type glycans and said cells can be grown at large scale (Jones et al, 2003; Nichols et al, 2002). The present invention teaches that these cells can efficiently produce an additional class of immunoglobulins having different characteristics. It was unexpectedly found that IgA can be produced in PER.C6 cells at levels comparable to those for IgG.

To obtain expression of nucleic acid sequences encoding IgA, it is well known to those skilled in the art that sequences capable of driving such expression can be functionally linked to the nucleic acid sequences encoding the IgA molecules, resulting in recombinant nucleic acid molecules encoding an IgA in expressible format. Functionally linked is meant to describe that the nucleic acid sequences encoding the IgA antibody fragments or precursors thereof are linked to the sequences capable of driving expression such that these sequences can drive expression of the antibodies or precursors thereof. Useful expression vectors are available in the art, e.g. the pcDNA vector series of Invitrogen. Where the sequence encoding the IgA polypeptide of interest is properly inserted with reference to sequences governing the transcription and translation of the encoded polypeptide, the resulting expression cassette is useful to produce the IgA of interest, referred to as expression. Sequences driving expression may include promoters, enhancers and the like, and combinations thereof. These should be capable of functioning in the host cell, thereby driving expression of the nucleic acid sequences that are functionally linked to them. Promoters can be constitutive or regulated, and can be obtained from various sources, including viruses, prokaryotic, or eukaryotic sources, or artificially designed. Expression of nucleic acids of interest may be from the natural promoter or derivative thereof or from an entirely heterologous promoter. Some well-known and much used promoters for expression in eukaryotic cells comprise promoters derived from viruses, such as adenovirus, e.g. the E1A promoter, promoters derived from cytomegalovirus (CMV), such as the CMV immediate early (IE) promoter, promoters derived from Simian Virus 40 (SV40), and the like. Suitable promoters can also be derived from eucaryotic cells, such as methallothionein (MT) promoters, elongation factor 1α (EF-1α) promoter, actin promoter, an immunoglobulin promoter, heat shock promoters, and the like. In one embodiment the sequence capable of driving expression comprises a region from a CMV promoter, preferably the region comprising nucleotides -735 to +95 of the CMV immediate early gene enhancer/promoter. This gives particularly high expression levels in cells expressing E1A of an adenovirus.

Culturing a cell is done to enable it to metabolize, and/or grow and/or divide and/or produce recombinant proteins of interest. This can be accomplished by methods well known to persons skilled in the art, and includes but is not limited to providing nutrients for the cell. The methods comprise growth adhering to surfaces, growth in suspension, or combinations thereof. Several culturing conditions can be optimized by methods well known in the art to optimize protein production yields. Culturing can be done for instance in dishes, roller bottles or in bioreactors, using batch, fed-batch, continuous systems, hollow fiber, and the like. In order to achieve large scale (continuous) production of recombinant proteins through cell culture it is preferred in the art to have cells capable of growing in suspension, and it is preferred to have cells capable of being cultured in the absence of animal- or human-derived serum or animal- or human-derived serum components. Thus purification is easier and safety is enhanced due to the absence of additional animal or human proteins derived from the culture medium, while the system is also very reliable as synthetic media are the best in reproducibility.
The conditions for growing or multiplying cells (see e.g. Tissue Culture, Academic Press, Kruse and Paterson, editors (1973)) and the conditions for expression of the recombinant product may differ somewhat, and optimization of the process is usually performed to increase the product yields and/or growth of the cells with respect to each other, according to methods generally known to the person skilled in the art. In general, principles, protocols, and practical techniques for maximizing the productivity of mammalian cell cultures can be found in Mammalian Cell Biotechnology: a Practical Approach (M. Butler, ed., IRL Press, 1991).

The IgA is expressed in the cells according to the invention, and may be recovered from the cells or preferably from the cell culture medium, by methods generally known to persons skilled in the art. Such methods may include precipitation, centrifugation, filtration, size-exclusion chromatography, affinity chromatography, cation- and/or anion-exchange chromatography, hydrophobic interaction chromatography, and the like.

It is demonstrated herein that IgA can be expressed at high levels without the necessity for first amplifying the nucleic acid sequences encoding the IgA within the host cells. This has the advantage that no large copy numbers are required for efficient expression according to the invention, in contrast to previously described recombinant IgA production systems, where amplification was required to obtain levels of around 20 pg IgA per cell per day. PER.C6 cells expressing IgG at high levels have been shown to contain usually between 1 and 10 copies of the nucleic acid encoding the IgG per cell (Jones et al, 2003). Methods to determine copy numbers are known to the person skilled in the art of molecular biology, and include Southern blotting, quantitative PCR, fiber-FISH, and the like. Hence, the invention provides for a method according to the invention wherein the cells comprise 1-20, usually between 1 and 10 copies per cell of the recombinant nucleic acid encoding the IgA molecule. This has the advantage of fast expression, as no labour-intensive and time consuming amplification step is needed to obtain clones with sufficiently high expression levels for analysis. Moreover, such cells are expected to be more stable than cells containing high copy numbers of the transgene, that are reported to display instability upon propagation of the cells (Kim et al, 1998; Barnes et al, 2003). Therefore, also in view of regulatory requirements the cells and methods according to the invention are an improvement over those of the prior art. Preferably, cells in the method of the invention express at least 5 pg IgA per seeded cell per day, more preferably at least 20 pg per seeded cell per day, still more preferably ate least 40 pg per seeded cell per day, and still more preferably at least 50 pg per seeded cell per day. Levels up to 60 pg per seeded cell per day are herein reported.

The IgA production system of the invention is not dependent upon co-expression of the J-chain for production of functional IgA. Optionally however, J-chain may be coexpressed.

An IgA molecule is an immunoglobulin wherein the heavy chains are alpha chains. An IgA molecule can have a monomeric or dimeric structure. An IgA molecule according to the invention may be of any origin, including human, rodent, chimeric, humanized, and the like, however human IgAs are preferred in the invention. Using a human cell line for the production provides these molecules with a human-type glycosylation, resulting in production of IgA molecules that are not recognized as foreign by the human immune system, because both the polypeptide and the glycan portion are human. The person skilled in the art will be aware of the possibilities to obtain human IgA sequences. The sequences for the constant regions are known, and are also provided herein. Sequences encoding human variable regions may e.g. be obtained by known methods such as phage display (methods e.g. described in CF Barbas III et al, Phage Display. A laboratory manual. Cold Spring Harbor Laboratory Press, Cold Spring Harbor, New York, 2001) or by immunizing transgenic mice that comprise genetic material encoding a human immunoglobulin repertoire (Fishwild et al, 1996; Mendez et al, 1997).

Antibodies may be used as naked molecules, or they may be in the form of immunoconjugates or labelled molecules, and so used as a magic bullet to deliver a cargo to a tumor or infection for therapy or imaging (Carter, 2001; Borrebaeck and Carlsson, 2001; Park and Smolen, 2001). Immunoconjugates comprise antigen binding domains and a non-antibody part such as a toxin, a radiolabel, an enzyme, and the like. IgA molecules may be labelled in the same way as IgG molecules. Hence, the term IgA molecule as used herein includes naked IgA molecules, but may also refer to immunoconjugates comprising IgA molecules.

The IgA generated in this study is against the human tumour antigen EpCAM (epithelial cell adhesion molecule), a 40kDa glycoprotein expressed on the surface of colon carcinoma cells. The high expression of EpCAM on colon carcinomas makes it an attractive target for immunotherapy. The antibody was isolated from a semi-synthetic phage library as a single chain Fv fragment named UBS54 (WO 01/48485; Huls et al, 1999b). In one aspect the invention therefore provides a recombinant human IgA molecule that is capable of binding to EpCAM. In other embodiments said human IgA molecules are used for the preparation of a medicament and/or for direct treatment of a disease such as cancer.

### EXAMPLES

The invention will now be described by some examples, not to be construed to limit the scope of the invention. The practice of this invention will employ, unless otherwise indicated, conventional techniques of immunology, molecular biology, microbiology, microbiology, cell biology, and recombinant DNA, which are within the skill of the art. See e.g. Sambrook, J. and Russell, D.W. (2001). Molecular cloning: a laboratory manual. Pub. Cold Spring Harbor Laboratory Press; Current Protocols in Molecular Biology, Ausubel FM, et al, eds, 1987; the series Methods in Enzymology (Academic Press, Inc.); PCR2: A Practical Approach, MacPherson MJ, Hams BD, Taylor GR, eds, 1995; Antibodies: A Laboratory Manual, Harlow and Lane, eds, 1988.

### Example 1. Construction of pEpcamIgA expression vector.

An expression plasmid was generated which encodes both the light and heavy chains of an IgA antibody that recognizes EpCAM. The DNA encoding the antigen-binding region of this antibody was first isolated from a scFv phage display library (Huls et al, 1999). A leader sequence and constant regions were added essentially as described in Boel et al, 2000. The genomic DNA encoding the light and heavy chains (genomic sequences of the antibody-encoding regions provided in SEQ. ID. NOs. 1 and 2, amino acid sequences of the encoded anti-EpCAM IgA provided in SEQ. ID. NOs. 3 and 4) were then amplified using PCR to append convenient restriction sites, and then cloned into the expression vector pcDNA3002(Neo). The following primers were used for PCR of the light chain:

E001: CCTGGCGCGCCACCATGGCATGCCCTGGCTTCCTGTGG (SEQ. ID. NO. 5)

E002: CCGGGTTAACTAACACTCTCCCCTGTTGAAGC (SEQ. ID. NO. 6).

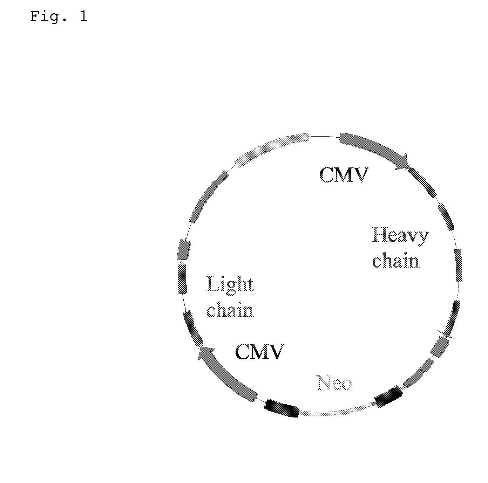

The following primers were used for PCR of the heavy chain: E003: GGAGGATCCGCCACCATGGCATGCCCTGGCTTCCTGTGG (SEQ. ID. NO. 7) E003: GGAGGATCCGCCACCATGGCATGCCCTGGCTTCCTGTGG (SEQ. ID. NO. 7) P01: GGACCGCTAGCTCAGTAGCAGGTGCCGAC (SEQ. ID. NO. 8). The start codon (E001, E003) and stop codon (E002, P01) are in bold. Restriction sites AscI (E001), HpaI (E002), BamHI (E003) and NheI (P01) are underlined. Primers E001 and E003 also include a Kozak sequence (italics). The light chain fragment of approximately 0.9 kb was digested with AscI-HpaI and inserted into pcDNA3002(Neo) digested with the same enzymes. The heavy chain fragment of approximately 2.0 kb was then digested with BamHI-NheI and inserted into pcDNA3002(Neo) containing the light chain digested with the same enzymes. The resulting plasmid is pEpcamIgA (Fig. 1). The generated construct contains DNA encoding a kappa light chain and an alpha1 heavy chain, both preceded by a CMV promoter. The expression vector pcDNA3002(Neo), which has been described in international patent application PCT/NL02/00841, was deposited on December 13, 2001 at the European Collection of Cell Cultures (ECACC) under number 01121318.

### Example 2. Transfection of PER.C6™ cell line and production of IgA.

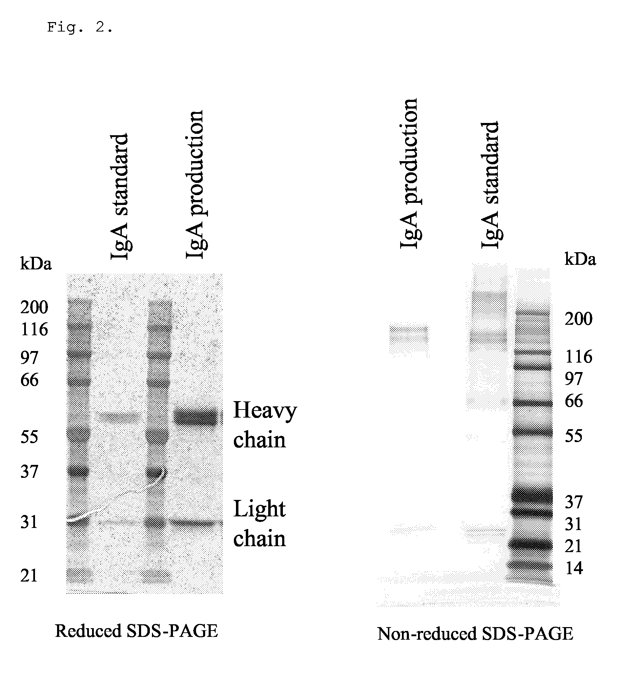

Cells were transfected with pEpcamIgA by a lipofectamine based method. In brief, PER.C6™ cells were seeded at 3.5 x 10⁶ cells per 96mm tissue culture dish. For each dish, 2µg plasmid DNA was mixed with 10µl lipofectamine (Gibco); this was added to the cells in serum free DMEM medium (total volume 7ml) and incubated for 4 hours. This was then replaced with DMEM medium (i.e. containing serum). The following day (and for the ensuing 3 weeks) cells were grown in DMEM medium in the presence of 0.5mg/ml Geneticin (G418) to select for clones that were stably transfected with the plasmid. Clones were tested for IgA productivity by ELISA analysis. In brief, cells were plated at 0.5 x 10⁶ cells per well of a 6-well dish in DMEM serum. These were incubated for 4 days, after which time supernatant was harvested and IgA concentration measured. For ELISA analysis, wells of a 96-well plate were coated with antibody raised against Ig kappa light chain. After blocking with a BSA solution, samples were added to wells at varying dilutions and incubated for 1 hour. The standard used was human IgA (Accurate Chemical cat. YSRTPHP002). After washing, detection antibody (HRP-labelled anti-IgA) was applied for 30 minutes. After a further washing step, substrate O-phenylene diamine dihydrochloride was added. Antibody concentration was determined by comparing optical density at 492nm with that of the known antibody standard. The highest-producing clones produced around 60 pg IgA/seeded cell/day. This is comparable to results seen when IgG-producing cell lines are measured with the same analysis.
Production of antibody was performed in serum-free medium. Thus the adherent cells in tissue culture flasks were transferred to roller bottles in PER.C6™ suspension growth medium (under which conditions the cells grow in suspension). After one week, supernatant was harvested and electrophoresed on reducing and non-reducing SDS-PAGE (Fig. 2). The reducing gel indicates that IgA is produced efficiently, with essentially all protein either intact light or intact heavy chain. The heavy chains of both the anti-Epcam sample and the standard IgA run as a doublet; the reason for this is unclear. The non-reducing gel shows that the IgA has a molecular weight of between 116 and 200kDa; this shows that the species produced is monomeric, and no evidence of high molecular weight aggregated material or low molecular weight fragments are visible. Again the protein runs as a doublet; the control material is also present as a doublet, but also contains many unidentified bands.

The examples above demonstrate that PER.C6™ cells may be transfected with a plasmid expressing IgA to give cells with a high IgA productivity.

### REFERENCES

Barnes, L.M., Bentley, C.M., Dickson, A.J. (2003). Stability of protein production from recombinant mammalian cells. Biotechnol. and Bioeng. 81, 631-639.

Berdoz, J., Blanc, C.T., Reinhardt, M., Kraehenbuhl, J., Corthesy, B. (1999). In vitro comparison of the antigen-binding and stability properties of the various molecular forms of IgA antibodies assembled and produced in CHO cells. Proc Natl Acad Sci U S A. 96, 3029-34.

Boel, E., Verlaan, S., Poppelier, M.J., Westerdaal, N.A., Van Strijp, J.A., Logtenberg, T. (2000). Functional human monoclonal antibodies of all isotypes constructed from phage display library-derived single-chain Fv antibody fragments. J Immunol Methods. 239, 153-66.

Borrebaeck, C.A.K. and Carlsson, R. (2001). Human therapeutic antibodies. Curr. Op. Pharmacol. 1, 404-408.

Carter, P. (2001). Improving the efficacy of antibody-based cancer therapies. Nature Reviews: Cancer 1, 118-129.

Chintalacharuvu, K.R., Morrison, S.L. (1999). Production and characterization of recombinant IgA. Immunotechnol. 4, 165-174.

Corthesy, B. (2002). Recombinant immunoglogulin A: powerful tools for fundamental and applied research. Trends in Biotechnol. 20, 65-71.

Dechant, M. and Valerius, T. (2001). IgA antibodies for cancer therapy. Crit. Rev. in Oncol./Haematol. 39, 69-77.

van Egmond, M., van Spriel, A.B., Vermeulen, H., Huls, G., van Garderen, E., van de Winkel, J.G. (2001). Enhancement of polymorphonuclear cell-mediated tumor cell killing on simultaneous engagement of fcgammaRI (CD64) and fcalphaRI (CD89). Cancer Res. 61, 4055-4060.

Fishwild, D.M., O'Donnell, S.L., Bengoechea, T., Hudson, D.V., Harding, F., Bernhard, S.L., Jones, D., Kay, R.M., Higgins, K.M., Schramm, S.R., Lonberg, N. (1996). High-avidity human IgG kappa monoclonal antibodies from a novel strain of minilocus transgenic mice. Nat Biotechnol. 14, 845-51. Cancer Immunol Immunother. 30, 43-50.

Ghetie, M.A., Podar, E.M., Ilgen, A., Gordon, B.E., Uhr, J.W., Vitetta, E.S. (1997). Homodimerization of tumor-reactive monoclonal antibodies markedly increases their ability to induce growth arrest or apoptosis of tumor cells. Proc Natl Acad Sci U S A. 94, 7509-14.

Huls, G., Heijnen, I.A., Cuomo, E., van der Linden, J., Boel, E., van de Winkel, J.G., Logtenberg, T. (1999a). Antitumor immune effector mechanisms recruited by phage display-derived fully human IgG1 and IgA1 monoclonal antibodies. Cancer Res. 59, 5778-5784.

Huls, G.A., Heijnen, I.A.F.M., Cuomo, M.E., Koningsberger, J.C., Wiegman, L., Boel, E., van der Vuurst-de Vries. A-R., Loyson, S.A.J., Helfrich, W., van Berge Henegouwen, G.P., van Meijer, M., de Kruif, J., Logtenberg, T. (1999b). A recombinant, fully human monoclonal antibody with antitumor activity constructed from phage-displayed antibody fragments. Nat Biotechnol. 17, 276-281.

Johansen F.E., Braathen R., Brandtzaeg P. (2000). Role of J chain in secretory immunoglobulin formation. Scand. J. Immunol. 52, 240-248.

Johansen, F.E., Braathen, R., Brandtzaeg, P. (2001). The J chain is essential for polymeric Ig receptor-mediated epithelial transport of IgA. J Immunol. 167, 5185-5192.

Jones, D., Kroos, N., Anema, R., Van Montfort, B., Vooys, A., Van Der Kraats, S., Van Der Helm, E., Smits, S., Schouten, J., Brouwer, K., Lagerwerf, F., Van Berkel, P., Opstelten, D-J., Logtenberg, T., Bout, A. (2003). High-level expression of recombinant IgG in the human cell line PER.C6™. Biotechnol Prog. 19, 163-8.

Kim, S.J., Kim, N.S., Ryu, C.J., Hong, H.J., Lee, G.M. (1998). Characterization of chimeric antibody producing CHO cells in the course of dihydrofolate reductase-mediated gene amplification and their stability in the absence of selective pressure. Biotechnol Bioeng 58, 73-84.

Longo, D.L. (2002). DR's orders: human antibody kills tumors by direct signalling. Nat. Med. 8, 781-783.

Mattu, T.S., Pleass, R.J., Willis, A.C., Kilian, M., Wormald, M.R., Lellouch, A.C., Rudd, P.M., Woof, J.M., Dwek, R.A. (1998). The glycosylation and structure of human serum IgA1, Fab, and Fc regions and the role of N-glycosylation on Fc alpha receptor interactions. J. Biol. Chem. 273, 2260-72.

Mendez, M.J., Green, L.L., Corvalan, J.R., Jia, X.C., Maynard-Currie, C.E., Yang, X.D., Gallo, M.L., Louie, D.M., Lee, D.V., Erickson, K.L., Luna, J., Roy, C.M., Abderrahim, H., Kirschenbaum, F., Noguchi, M., Smith, D.H., Fukushima, A., Hales, J.F., Klapholz, S., Finer, M.H., Davis, C.G., Zsebo, K.M., Jakobovits, A. (1997). Functional transplant of megabase human immunoglobulin loci recapitulates human antibody response in mice. Nat Genet. 15, 146-56.

Nichols, W.W., Lardenoije, R., Ledwith, B.J., Brouwer, K., Manam, S., Vogels, R., Kaslow, D., Zuidgeest, D., Bett, A.J., Chen, L., van der Kaaden, M., Galloway, S.M., Hill, R.B., Machotka, S.V., Anderson, C.A., Lewis, J., Martinez, D., Lebron, J., Russo, C., Valerio, D. and Bout, A. Propagation of adenoviral vectors: use of PER.C6 cells. In *Adenoviral vectors for gene therapy,* (Ed. Curiel D.T. and Douglas, J.T.). Pub. Academic Press, 2002.

Novak, J., Tomana, M., Kilian, M., Coward, L., Kulhavy, R., Barnes, S., Mestecky, J. (2000). Heterogeneity of O-glycosylation in the hinge region of human IgA1. Mol Immunol. 37, 1047-56.

Park, J.W. and Smolen, J. (2001). Monoclonal antibody therapy. Adv. Prot. Chem. 56, 369-421.

van Spriel, A.B., van den Herik-Oudijk, I.E., van Sorge, N.M., Vile, H.A., van Strijp, J.A., van de Winkel, J.G. (1999). Effective phagocytosis and killing of Candida albicans via targeting FcgammaRI (CD64) or FcalphaRI (CD89) on neutrophils. J Infect Dis. 179, 661-669.

Tutt, A.L., French, R.R., Illidge, T.M., Honeychurch, J., McBride, H.M., Penfold, C.A., Fearon, D.T., Parkhouse, R.M.E., Klaus, G.G.B. and Glennie, M.J. (1998). Monoclonal antibody therapy of B cell lymphoma: signalling activity on tumour cells appears more important than recruitment of effectors. J. Immunol. 161, 3176-3185.

Vidarsson, G., van der Pol, W.L., van den Elsen, J.M., Vile, H., Jansen, M., Duijs, J., Morton, H.C., Boel, E., Daha, M.R., Corthesy, B., van de Winkel, J.G. (2001). Activity of human IgG and IgA subclasses in immune defense against Neisseria meningitidis serogroup B. J Immunol. 166, 6250-6256.

Yoo, E.M., Chintalacharuvu, K.R., Penichet, M.L., Morrison, S.L. (2002). Myeloma expression systems. J. Immunol. Meths. 261, 1-20.

## Claims

1. A cell expressing E1A and E1B proteins of an adenovirus, wherein said cell comprises recombinant nucleic acid encoding an IgA molecule in expressible format.

2. A cell according to claim 1, wherein said cell is a human cell.

3. A cell according to any of the aforegoing claims, wherein said cell is derived from a retina cell.

4. A cell according to any of the aforegoing claims, wherein said cell is derived from a primary cell.

5. A cell according to any of the aforegoing claims, wherein said cell is derived from a PER.C6™ cell.

6. A cell according to any of the aforegoing claims, wherein said cell comprises 1-20 copies of said recombinant nucleic acid encoding the IgA molecule.

7. A cell according to any of the aforegoing claims, wherein said IgA molecule is a human IgA molecule.

8. A cell according to any of the aforegoing claims, wherein said IgA molecule has a constant region comprising amino acids 137 to 489 of SEQ. ID. NO. 3.

9. A cell according to any of the aforegoing claims, wherein said cell, when seeded at 0.5 x 10⁶ cells/well and cultured in 6-well tissue culture plates at 37°C in DMEM with 10% serum under an atmosphere containing 10% CO₂, produces at least 5 pg IgA/seeded cell/day.

10. A cell according to claim 9, wherein said cell, when seeded at 0.5 x 10⁶ cells/well and cultured in 6-well tissue culture plates at 37°C in DMEM with 10% serum under an atmosphere containing 10% CO₂, produces at least 20 pg IgA/seeded cell/day.

11. A cell according to claim 10, wherein said cell, when seeded at 0.5 x 10⁶ cells/well and cultured in 6-well tissue culture plates at 37°C in DMEM with 10% serum under an atmosphere containing 10% CO₂, produces at least 40 pg IgA/seeded cell/day.

12. A method for recombinant production of an IgA molecule, said method comprising culturing a cell according to any of the aforegoing claims and expressing said recombinant nucleic acid encoding an IgA.

13. Use of a cell expressing E1A and E1B proteins of an adenovirus for recombinant expression of IgA molecules.
